# EUROPEAN PATENT SPECIFICATION

(11) **EP 3 146 900 B1**
(45) Date of publication and mention of the grant of the patent: **29.04.2020**
(21) Application number: 15796933.8
(22) Date of filing: 26.05.2015
(51) Int. Cl.: A61B 6/00, A61B 6/03, G01R 33/565, H04N 19/51

(54) **MEDICAL IMAGE PHOTOGRAPHING APPARATUS AND MEDICAL IMAGE CORRECTION METHOD USING DEPTH CAMERA**
VORRICHTUNG ZUM FOTOGRAFIEREN MEDIZINISCHER BILDER UND VERFAHREN ZUR KORREKTUR MEDIZINISCHER BILDER MIT VERWENDUNG EINER TIEFENKAMERA
APPAREIL DE PHOTOGRAPHIE D'IMAGES MÉDICALES ET PROCÉDÉ DE CORRECTION D'IMAGES MÉDICALES À L'AIDE D'UNE CAMÉRA DE PROFONDEUR

(30) Priority: 23.05.2014 KR 20140062011
(43) Date of publication of application: 29.03.2017
(73) Proprietor: Vatech Co., Ltd., Hwaseong-si, Gyeonggi-do 445-170 (KR); Vatech Ewoo Holdings Co., Ltd., Hwaseong-si, Gyeonggi-do 445-170 (KR)
(72) Inventor: KIM, Jae Chul, Hwaseong-si Gyeonggi-do 445-170 (KR); JANG, Seong Eun, Hwaseong-si Gyeonggi-do 445-170 (KR)
(74) Representative: Markfort, Iris-Anne Lucie
(86) International application number: PCT/KR2015/005244
(87) International publication number: WO 2015/178745

(56) References cited:
- WO-A1-2014/038603
- JP-A- 2008 188 164
- JP-A- 2008 188 164
- JP-A- 2010 115 429
- JP-A- 2010 115 429
- JP-A- 2013 158 372
- KR-A- 20130 028 057
- US-A1- 2005 171 420
- US-A1- 2012 170 824
- US-A1- 2013 281 818
- US-A1- 2014 161 222
- OLESEN OLINE V ET AL: "DLP technology application: 3D head tracking and motion correction in medical brain imaging", VISUAL COMMUNICATIONS AND IMAGE PROCESSING; 20-1-2004 - 20-1-2004; SAN JOSE,, vol. 8979, 7 March 2014 (2014-03-07), pages 897904-897904, XP060034741, DOI: 10.1117/12.2035374 ISBN: 978-1-62841-730-2 & CARSON R E ET AL: "Design of a motion-compensation OSEM list-mode algorithm for resolution-recovery reconstruction for the HRRT", 2003 IEEE NUCLEAR SCIENCE SYMPOSIUM CONFERENCE RECORD. / 2003 IEEE NUCLEAR SCIENCE SYMPOSIUM AND MEDICAL IMAGING CONFERENCE. PORTLAND, OR, OCT. 19 - 25, 2003; [IEEE NUCLEAR SCIENCE SYMPOSIUM CONFERENCE RECORD], NEW YORK, NY : IEEE, US, 19 October 2003 (2003-10-19), page 3281, XP010740407, ISBN: 978-0-7803-8257-2

## Description

### Technical Field

The present invention relates, generally to radiographing apparatus and method of correcting a medical radiograph.

### Background Art

A conventional method of correcting a medical radiograph is as follows.

A reference document of International Publication No. WO 2013/162201 A1 (published on 31 October 2013 entitled "METHOD FOR TRACKING MOTION OF SUBJECT IN REAL TIME AND FOR CORRECTING MEDICAL IMAGE") discloses a conventional method of correcting a medical radiograph.

In the above document, a medical radiograph is corrected by outputting real-time movement information of a subject captured by a movement detection module; converting and outputting the real-time moving information to three-axis parameters by a movement calculation module; operating an operating motor by an operation module in response to the three-axis parameters; and moving by a distance that the subject has actually moved in response to the operation of the operating motor by a medical image data obtaining module.

In addition, in some conventional techniques, a post image processing method that reconstructs a radiographed image at first, and then performs an image processing is used.

However, the above document is problematic when an equipment vibration, or a positional change of a subject is small thus the radiographing device is not repositioned. For example, a radiograph is degraded when a positional change of a subject is small, whereby the X-ray radiographing device is not repositioned and thus the image correction is not performed.

In addition, a radiographing time delay is caused since a movement of the X-ray radiographing device and a data acquisition of the medical radiograph are not performed in real time. Further, when the movement of the X-ray radiographing device or obtained movement information is incorrect, a completely different radiograph may be obtained.

Further, in a conventional technique using a post image processing method, original data may be damaged since an image correction is performed after performing a reconstruction algorithm.

Meanwhile, in the medical field, X-ray radiographs such as CTs are used for diagnoses purposes. However, X-ray radiographs are images of internal human organs and are unfamiliar to ordinary persons who are not medical workers. In addition, in dental clinics and in the plastic surgery field, there is a request for checking a dimensional change in a surface of the body such as face after a procedure; however, the X-ray radiograph alone is not enough to meet such a requirement.

Therefore, there is a need for developing an apparatus for a medical radiograph, the apparatus being capable of clearly radiographing a 3D surface.

US 2005/171420 A1 relates to a method and also an imaging system to compensate for patient motion when recording a series of images in medical imaging, in which a number of images of an area under examination of a patient are recorded at intervals with an imaging system and are related to one another.

US 2013/281818 A1 discloses a scanner configured to generate scan data of a patient volume or area, a processor configured to implement control operations, the control operations being directed to acquisition of the scan data or to processing of the scan data, and a monitoring system including a range imaging camera positioned for a field of view such that the monitoring system is configured to capture spatial data indicative of movement of an object spaced from the scanner. The processor is configured to implement an adjustment in the control operations based on the spatial data.

### Technical Problem

One of the objects of the present invention is solve the problems of the prior art as described above.

Therefore, an embodiment of the present invention provides a radiographing device for a medical radiograph in which a clear 3D surface radiograph is obtained by using a depth camera.

Other objects and advantages of the present invention that are not mentioned above can be understood by the following description, and become apparent with reference to the embodiments of the present invention. Also, it is obvious to those skilled in the art to which the present invention pertains that the objects and advantages of the present invention can be realized by the means as claimed and combinations thereof.

### Technical Solution

According to one aspect of an embodiment of the present invention, there is provided a radiographing apparatus for a medical radiograph with the features of claim 1.
According to another aspect of an embodiment of the present invention, there is provided a method of correcting a medical radiograph with the features of claim 5.

### Advantageous Effects

According to the above embodiment of the present invention, a damaged radiograph caused by a vibration of an equipment of by a movement of a patient while radiographing is corrected is performed based on original data, and not based on a reconstructed radiograph. Thus, a precise correction may be obtained.

According to the embodiment of the present invention, a radiographing time delay does not occur since the acquisition of medical radiograph data and the correction of the medical radiograph are performed in real time.

According to the embodiment of the present invention, the radiographing device is not moved to correct the radiograph, and thus the radiograph is corrected even a small movement of the subject compared to the conventional technique.

According to the embodiment of the present invention, a depth surface image of the subject is obtained by using the depth camera. The depth surface image is composed with a medical radiograph (for example, an X-ray CT radiograph) obtained from the X-ray radiographing device such that a clear 3D surface radiograph of the subject 10 is obtained.

### Description of Drawings

FIG. 1 is a perspective view showing a radiographing apparatus for a medical radiograph using a depth camera according to an embodiment of the present invention.
FIG. 2 is a block diagram showing a configuration of a computed tomography (CT) apparatus including a medical radiograph correction device for implementing a method of correcting a medical radiograph by using a depth camera according to an embodiment of the present invention.
FIG. 3 is a flowchart explaining a method of correcting a medical radiograph by using a depth camera according to an embodiment of the present invention.
FIG. 4 is a flowchart explaining a process of correcting a medical radiograph performed in a correction unit according to an embodiment of the present invention.
FIG. 5 is a block diagram showing a configuration of radiographing apparatus for a medical radiograph for implementing a method of a medical radiograph by using a depth camera according to an embodiment of the present invention.

### Mode for Invention

The objects, features, and advantages of the invention will become apparent through the exemplary embodiments that are illustrated in the accompanying drawings and detailed in the following description. Accordingly, the inventive technological concept can be made by those skilled in the art without departing from the scope of the invention. In the following explanations, when a detailed description of well-known functions or configurations is determined to unnecessarily cloud the gist of the present invention, the detailed description thereof will be omitted. Terminology described below is defined considering functions in the present invention and may vary according to a user's or operator's intention or usual practice. Thus, the meanings of the terminology should be interpreted based on the overall context of the present specification. Hereinafter, exemplary embodiments of the present invention will be described in detail below with reference to the accompanying drawings.

Throughout the specification, when an element "includes" another element, it means that the element excludes other elements but may further include other elements as long as the opposite description is not particularly made.

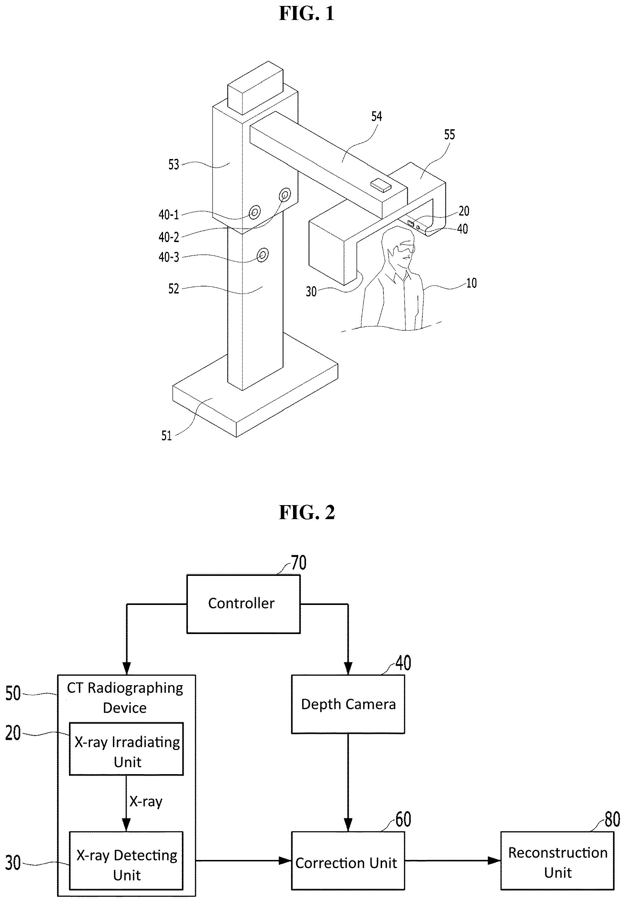

FIG. 1 is a view for explaining a computed tomography (CT) radiographing device according to an embodiment of the present invention for explaining a concept of a method of correcting a medical radiograph by using a depth camera, and FIG. 2 is a block diagram showing a configuration of a CT radiographing device with a medical radiograph correction device for implementing the method of correcting the medical radiograph by using the depth camera according to the embodiment of the present invention.

Referring to FIGS. 1 and 2, an apparatus for correcting a medical radiograph will be described.

The CT radiographing device 50 for the medical radiograph according to the present invention includes: a base 51 installed on a ground surface; a column 52 standing up from the base 51; an elevating unit 53 capable of moving in vertical directions along the column 52; a rotation arm supporting unit 54 extending from the elevating unit 53 in a horizontal direction; and a rotation arm 55 rotatably provided in a lower part of the rotation arm supporting unit 54.

The radiographing apparatus for the medical radiograph according to an embodiment of the present invention includes an X-ray radiographing device radiographing a subject 10 and obtaining a medical radiograph data of the subject 10, and a first depth camera 40 provided in the radiographing apparatus and measuring a movement of the subject 10.

In the embodiment of the present invention, the CT radiographing device 50 is used as an example of the X-ray radiographing device, and CT data is used as an example of medical radiograph data.

The CT radiographing device 50 further includes an X-ray irradiating unit 20 irradiating X-rays and an X-ray detecting unit 30 detecting the X-rays passing through the subject 10. Herein, the X-ray irradiating unit 20 and the X-ray detecting unit 30 are respectively provided in both ends of the rotation arm 55 to face each other.

The first depth camera 40 refers to a camera that generates a depth data for each pixel of the radiographed subject 10 based on a time of flight (TOF) of the X-rays, the time of flight (TOF) is an elapsed time in which light irradiated from the depth camera 40 is returned by being reflected by the subject 10. The first depth camera 40 may include a light source (for example, LED) to irradiate light toward the subject 10.

The first depth camera 40 according to the embodiment of the present invention is provided in the rotation arm 50. However, it is not limited thereto and the first depth camera 40 may be provided in the column 52, or in the elevating unit 53. In other words, positions and number of the first depth camera 40 are not limited.

The medical radiograph correcting device according to the embodiment of the present invention is capable of controlling the CT radiographing device 50 and the first depth camera 40 such that the CT data and the depth data are obtained at the same time, and includes a controller 70 that controls the CT radiographing device 50 and the first depth camera 40 to transmit the obtained radiograph data and the depth data.

In addition, the medical radiograph correction device according to the embodiment of the present invention includes a correction unit 60 correcting a radiograph by using the radiograph data and the depth data that are transmitted from the CT radiographing device 50 and the first depth camera 40.

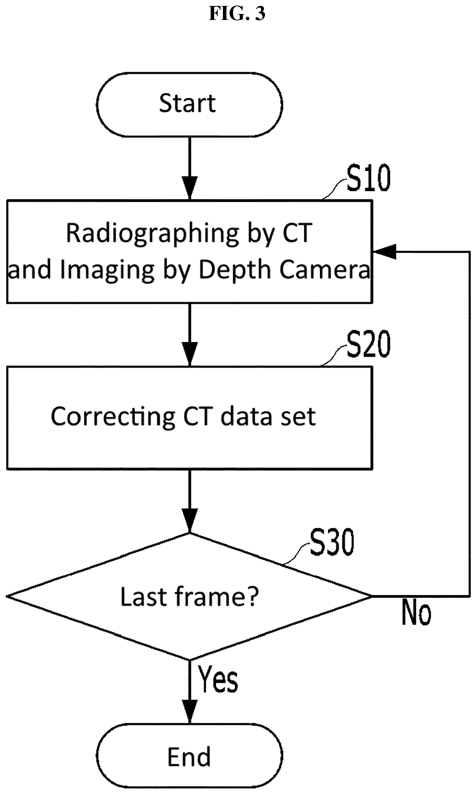

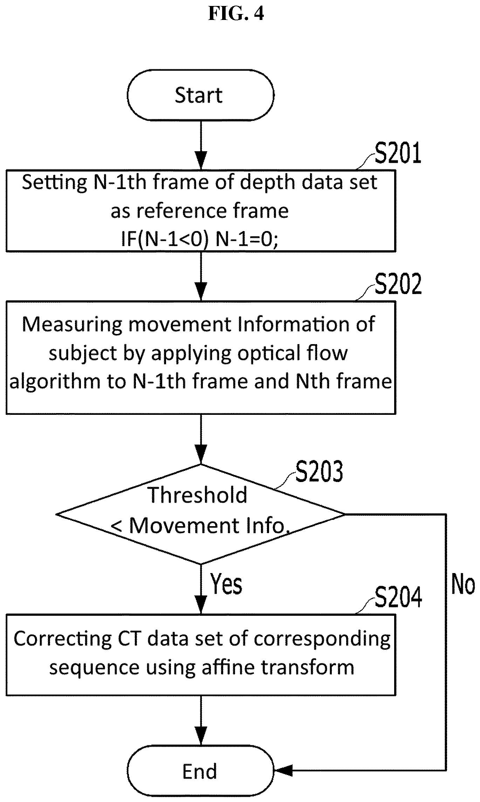

FIG. 3 is a flowchart explaining a method of correcting a medical radiograph by using a depth camera according to an embodiment of the present invention, and FIG. 4 is a flowchart explaining a process of correcting a medical radiograph performed in a correction unit according to an embodiment of the present invention.

Referring to FIGS. 1 to 4, the method of correcting the medical radiograph by using the depth camera is as follow.

The CT radiographing device 50 and the first depth camera 40 image the subject 10 (S10). In detail, the controller 70 controls the X-ray irradiating unit 20 to irradiate X-rays toward the subject 10, controls the X-ray detecting unit 30 to detect the X-rays that have passed through the subject 10, and obtains CT data of the subject 10. At the same time, the controller 70 controls the first depth camera 40 to image the subject 10 and obtains the depth data thereof. The CT radiographing device 50 and the first depth camera 40 transmit the obtained data to the correction unit 60.

The correction unit 60 corrects a CT radiograph by using the CT data and the depth data (S20).

When correcting the radiograph (S20), an N-1th frame of the depth data is set as a reference frame (S201).

Then, the movement information of the subject 10 is measured by comparing the reference frame (N-1th frame) to a current frame (S202). In other words, differences in pixel values between the reference frame and the current frame are calculated. A movement vector is calculated based on the calculated differences and is determined as movement information of the subject 10. The movement information may be measured in a pixel unit as described above. However, persons skilled in the art may know that the movement information may be measured in a macro-block unit. Herein, an optical flow algorithm may be used for calculating values of the motion vector. When a position of a camera is moved relative to the subject 10 or a position of the subject 10 is moved relative to the camera while obtaining temporally continuous radiographs, each pixel changes in its brightness and such a change in contrast is called an optical flow. A movement vector of two succeeding images of the subject 10 may be calculated by using optical flow information. In other words, movement information of the subject 10 is calculated. However, a calculation process is well known in the art, thus a detailed description of the calculating process is omitted in the present invention.

Then, the movement information is compared to a predetermined threshold (S203). Herein, it may be understood that the threshold may be changed by an experiment such that the threshold determines whether a radiograph correction is performed when the subject 10 has moved by some degree. A common threshold may be applied to all depth data, or a different threshold may be applied to each frame.

When the movement information exceeds the predetermined threshold, the corresponding radiograph data is corrected by applying an affine transform to the corresponding CT data based on the movement information calculated in S203 to (S204). Herein, a sequence in which a movement of the subject 10 occurs is known by previous information, for example, when time duration from the beginning of the radiograph to the end thereof, in other words, a total radiographing time, is assumed to be 600 seconds and 500 radiographs are radiographed during the total radiographing time. When movement of the subject 10 occurs at 300 seconds, the movement is known by the depth data. The CT radiograph is corrected by applying an affine transform to a 250^{th} radiograph that is radiographed at 300 seconds, in other words, CT data that temporally corresponds to a current frame (Nth frame) of the depth data.

The affine transform used when correcting the CT radiograph (S204) is a conventional method such as rotating, moving in parallel, scaling, shearing, etc. of an image. For example, when the movement information of the subject 10 measured in S202 includes a rotation about 45 degrees, a shift in x-axis by 5, and a shift in y-axis by 10, then, the above values may be substituted to an affine transform equation to correct the CT radiograph.

The affine transform is well known in the art and is used in various fields. Thus, a detailed description of transformation is omitted in the embodiment of the present invention.

Meanwhile, the CT radiographing device 50 and the first depth camera 40 repeatedly image the subject 10 (S10), and the CT data is repeatedly corrected (S20) until the last frame of the depth data in which the CT radiographing is finished is obtained (S30).

The CT data corrected by the method of correcting the medical radiograph according to the embodiment of the present invention may be reconstructed to a CT radiograph by using a conventional reconstruction algorithm by a reconstruction unit 80. Image quality of the reconstructed CT radiograph is better than that of a radiograph obtained by using a conventional method.

A described above, in the embodiment of the present invention, the CT radiographing device 50 is used as an X-ray radiographing device. Alternatively, a magnetic resonance imaging may be used as the X-ray radiographing device, thus the present is not limited thereto.

In addition, the optical flow algorithm and the affine transform are used as imaging processing techniques for correcting a medical radiograph; however, it is not limited thereto. In addition, the method of correcting a medical radiograph by using the depth camera according to the present invention as described above may be realized in the form of program instructions which may be implemented through various computer components, and may be recorded in a computer-readable storage medium. The computer-readable storage medium may include a program instruction, a data file, a data structure, and the like either alone or in combination thereof. The program instruction recorded in the computer-readable storage medium may be any program instruction particularly designed and structured for the present invention or known to those skilled in the field of computer software. Examples of the computer-readable storage medium include magnetic recording media such as hard disks, to floppy disks and magnetic tapes, optical data storage media such as CD-ROMs or DVD-ROMs, magneto-optical media such as floptical disks, and hardware devices, such as read-only memory (ROM), random-access memory (RAM), and flash memory, which are particularly structured to store and implement the program instruction. Examples of the program instruction include not only a mechanical language code formatted by a compiler but also a high level language code which may be implemented by a computer using an interpreter. The hardware devices may be configured to be operated by one or more software modules or vice versa to conduct the processes according to the present invention.

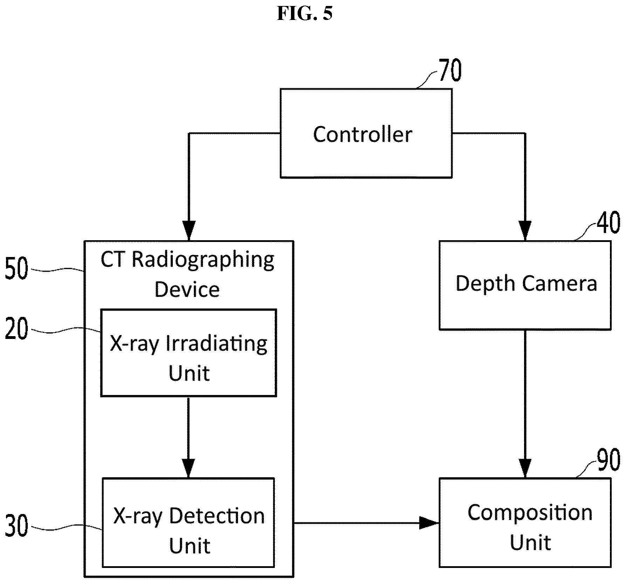

FIG. 5 is a block diagram showing a configuration of radiographing apparatus for a medical radiograph for implementing a method of a medical radiograph by using a depth camera according to an embodiment of the present invention.

Referring to FIG. 5, a method of obtaining a 3D surface radiograph by using the depth camera is as follows.

A second depth camera 40-1 and a third depth camera 40-2 are provided in front of the elevating unit 53 to face each other. The second depth camera 40-1 radiographs a first area of the subject 10 and obtains a depth surface image of the first area. The third depth camera 40-2 radiographs a second area of the subject 10 and obtains a depth surface image of the second area.

Herein, the second depth camera 40-1 and the third depth camera 40-2 may scan and radiograph the subject 10 by movement in upward and downward directions by the controller 70 since the second depth camera 40-1 and the third depth camera 40-2 are disposed in front of the elevating unit 53.

A fourth depth camera 40-3 is provided in front of the column 52, radiographs the other area of the subject 10, and obtains a depth surface image of the other area.

The radiographing apparatus according to the embodiment of the present invention includes three depth cameras 40-1, 40-2, and 40-3. However, it is not limited thereto. The depth camera may be provided in one, two, or more than four.

The depth cameras 40-1, 40-2, and 40-3 transmit the surface depth radiographs obtained from the subject 10 to a composition unit 90.

The CT radiographing device 50 radiographs the subject 10, obtains a medical radiograph (for example, X-ray CT radiograph) of the subject 10, and transmits the medical radiograph to the composition unit 90.

The composition unit 90 performs a composition by matching the transmitted depth surface images and the medical radiograph of the subject 10. A 3D depth surface image of the subject 10 is obtained by matching the radiographs. Herein, the method of composing a radiograph is well known in the art, thus detailed description thereof is omitted.

Particularly, according to the present invention, depth surface images obtained from the depth cameras 40-1, 40-2, and 40-3 are used for composing the radiograph, thus a clear 3D surface radiograph of the subject 10 may be generated.

Although the preferred embodiments of the present invention have been disclosed for illustrative purposes, those skilled in the art will appreciate that various modifications, additions and substitutions are possible, without departing from the scope of the invention as disclosed in the accompanying claims.

Therefore, the scope of the present invention shall not be limited to the above-described embodiments.

## Claims

1. A radiographing apparatus for a medical radiograph, the apparatus comprising:
an X-ray radiographing device radiographing a subject (10), and obtaining a medical radiograph data of the subject (10);
a depth camera (40) obtaining a depth data of the subject (10) including a reference frame and a current frame; and
a controller controlling the X-ray radiographing device and the depth camera to obtain the medical radiograph data and the depth data of the subject at the same time;
a correction unit (60) correcting the medical radiograph data transmitted from the X-ray radiographing device by using the depth data transmitted from the depth camera,
**characterized in that** the radiographing apparatus further comprises:
a composition unit (90) composing a depth surface image of the subject (10) by using the depth data and the medical radiograph data,
wherein the correction unit (60) calculates a movement information of the subject (10) by comparing two neighboring frames of the depth data including the reference frame and the current frame,
and corrects a part of the medical radiograph data corresponding to the current frame of depth data by applying the movement information to an affine transform formula when the movement information exceeds a predetermined threshold.

2. The radiographing apparatus of claim 1, further comprising:
a reconstruction unit (80) performing a radiographing reconstruction algorithm based on the corrected medical radiograph data.

3. The radiographing apparatus of claim 1, wherein the X-ray radiographing device includes:
a vertical column (52);
an elevating unit (53) capable of moving in vertical directions along the vertical column (52);
a rotation arm supporting unit (54) connected to the elevating unit (53); and
a rotation arm (55) connected to the rotation arm supporting unit (54), and including an X-ray irradiating unit (20) and an X-ray detecting unit (30) to face each other with the subject (10) interposed therebetween, and
wherein at least one depth camera (40) is provided in the rotation arm (55), in the vertical column (52), or in the elevating unit (53).

4. The radiographing apparatus of any of claims 1 to 3, wherein the movement information is a movement vector of two succeeding frames.

5. A method of correcting a medical radiograph, the method comprising:
radiographing a subject and obtaining a medical radiograph data of the subject by an X-ray radiographing device, and, at the same period, imaging the subject and obtaining a depth data of the subject by a depth camera (S10);
calculating a movement information of the subject by comparing two neighboring frames of the depth data, and correcting the medical radiograph data corresponding to the current frame of depth data using the calculated movement information by a correction unit (S20); and
performing a reconstruction algorithm based on the corrected medical radiograph data,
**characterized in that** the method further comprises:
composing a depth surface image of the subject by using the depth data and the medical radiograph data by a composing unit,
wherein the calculating a movement information includes:
setting an N-1th, where N is an integer, frame of the depth data as a reference frame (S201);
obtaining the movement information of the subject by comparing the N-1th reference frame of the depth data to a current Nth frame thereof (S202);
determining whether the movement information of the subject exceeds a predetermined threshold (S203); and
correcting a corresponding medical radiograph data by applying the movement information to an affine transform formula when the movement information exceeds the predetermined threshold (S204).

## Patentansprüche

1. Ein Röntgengerät für ein medizinisches Röntgenbild, wobei das Gerät umfasst:
ein Röntgen-Röntgengerät, das ein Subjekt (10) röntgt und ein medizinisches Röntgenbild des Subjekts (10) erhält;
eine Tiefenkamera (40), die Tiefendaten des Subjekts (10) einschließlich eines Bezugsrahmens und eines aktuellen Rahmens erhält; und
eine Steuereinheit (70), die das Röntgen-Röntgengerät und die Tiefenkamera (40) steuert, um gleichzeitig die medizinischen Röntgendaten und die Tiefendaten des Probanden (10) zu erhalten;
eine Korrektureinheit (60), die die von der Röntgen-Röntgenaufnahmeeinrichtung übertragenen medizinischen Röntgenbilddaten unter Verwendung der von der Tiefenkamera übertragenen Tiefendaten korrigiert,
**dadurch gekennzeichnet, dass** der Röntgenapparat ferner umfasst:
eine Zusammensetzungseinheit (90), die ein Tiefen-Oberflächenbild des Subjekts (10) unter Verwendung der Tiefendaten und der medizinischen Röntgenbilddaten zusammensetzt,
wobei die Korrektureinheit (60) eine Bewegungsinformation des Subjekts (10) berechnet, indem sie zwei benachbarte Bilder der Tiefendaten einschließlich des Bezugsbildes und des aktuellen Bildes vergleicht,
und korrigiert einen Teil der medizinischen Röntgenbilddaten, der dem aktuellen Rahmen der Tiefendaten entspricht, durch Anwendung der Bewegungsinformationen auf eine affine Transformationsformel, wenn die Bewegungsinformationen einen vorgegebenen Schwellenwert überschreiten.

2. Der Röntgenapparat nach Anspruch 1, der ferner umfasst:
eine Rekonstruktionseinheit (80), die einen Röntgenbild-Rekonstruktionsalgorithmus auf der Grundlage der korrigierten medizinischen Röntgenbilddaten ausführt.

3. Das Röntgengerät nach Anspruch 1, wobei das Röntgengerät Folgendes umfasst:
eine vertikale Säule (52);
eine Hebeeinheit (53), die in der Lage ist, sich in vertikalen Richtungen entlang der vertikalen Säule (52) zu bewegen;
eine Dreharm-Trageinheit (54), die mit der Hebeeinheit (53) verbunden ist; und
einen Dreharm (55), der mit der Dreharmträgereinheit (54) verbunden ist und eine Röntgenstrahlungseinheit (20) und eine Röntgendetektoreinheit (30) enthält, die einander gegenüberliegen, wobei das Objekt (10) dazwischen angeordnet ist, und
wobei mindestens eine Tiefenkamera (40) im Dreharm (55), in der vertikalen Säule (52) oder in der Hubeinheit (53) vorgesehen ist.

4. Das Röntgengerät nach einem der Ansprüche 1 bis 3, wobei die Bewegungsinformation ein Bewegungsvektor von zwei aufeinanderfolgenden Bildern ist.

5. Ein Verfahren zur Korrektur einer medizinischen Röntgenaufnahme, wobei das Verfahren umfasst:
Röntgenaufnahme eines Subjekts und Erhalten von medizinischen Röntgenbilddaten des Subjekts durch eine Röntgenaufnahmeeinrichtung und, in der gleichen Zeitspanne, Abbilden des Subjekts und Erhalten von Tiefendaten des Subjekts durch eine Tiefenkamera (S10);
Berechnen einer Bewegungsinformation des Subjekts durch Vergleichen zweier benachbarter Rahmen der Tiefendaten und Korrigieren der medizinischen Röntgenbilddaten entsprechend dem aktuellen Rahmen der Tiefendaten unter Verwendung der berechneten Bewegungsinformation durch eine Korrektureinheit (S20); und
Durchführung eines Rekonstruktionsalgorithmus auf der Grundlage der korrigierten medizinischen Röntgenbilddaten,
**dadurch gekennzeichnet, dass** die Methode ferner umfasst:
das Zusammensetzen eines Tiefen-Oberflächenbildes des Subjekts unter Verwendung der Tiefendaten und der medizinischen Röntgenbilddaten durch eine Zusammensetzungseinheit,
wobei die Berechnung einer Bewegungsinformation beinhaltet:
Setzen eines N-1. Rahmens, wobei N eine ganze Zahl ist, der Tiefendaten als Bezugsrahmen (S201);
Erhalten der Bewegungsinformation des Subjekts durch Vergleichen des N-1-ten Bezugsrahmens der Tiefendaten mit einem aktuellen N-ten Rahmen davon (S202);
Bestimmen, ob die Bewegungsinformation des Subjekts eine vorbestimmte Schwelle überschreitet (S203); und
Korrektur der entsprechenden medizinischen Röntgenbilddaten durch Anwendung der Bewegungsinformation auf eine affine Transformationsformel, wenn die Bewegungsinformation den vorgegebenen Schwellenwert (S204) überschreitet.

## Revendications

1. Un appareil de radiographie pour une radiographie médicale, l'appareil comprenant :
un appareil de radiographie radiographique pour radiographier un sujet (10), et obtenir des données de radiographie médicale du sujet (10) ;
une caméra de profondeur (40) obtenant des données de profondeur du sujet (10) comprenant un cadre de référence et un cadre courant ; et
un contrôleur (70) contrôlant l'appareil de radiographie et la caméra de profondeur (40) pour obtenir simultanément les données de la radiographie médicale et les données de profondeur du sujet (10) ;
une unité de correction (60) corrigeant les données radiographiques médicales transmises par l'appareil de radiographie à rayons X en utilisant les données de profondeur transmises par la caméra de profondeur,
**caractérisé en ce que** l'appareil de radiographie comprend en outre
une unité de composition (90) qui compose une image de surface en profondeur du sujet (10) en utilisant les données de profondeur et les données de la radiographie médicale,
dans lequel l'unité de correction (60) calcule une information de mouvement du sujet (10) en comparant deux trames voisines des données de profondeur comprenant la trame de référence et la trame courante,
et corrige une partie des données de la radiographie médicale correspondant au cadre actuel des données de profondeur en appliquant les informations de mouvement à une formule de transformation affine lorsque les informations de mouvement dépassent un seuil prédéterminé.

2. L'appareil de radiographie de la revendication 1, comprenant en outre :
une unité de reconstruction (80) effectuant un algorithme de reconstruction de radiographie basé sur les données de radiographie médicale corrigées.

3. L'appareil de radiographie selon la revendication 1, dans lequel l'appareil de radiographie comprend :
une colonne verticale (52) ;
une unité d'élévation (53) capable de se déplacer dans des directions verticales le long de la colonne verticale (52) ;
une unité de support de bras de rotation (54) reliée à l'unité élévatrice (53) ; et
un bras de rotation (55) relié à l'unité de support du bras de rotation (54), et comprenant une unité d'irradiation à rayons X (20) et une unité de détection à rayons X (30) pour se faire face avec le sujet (10) interposé entre elles, et
dans lequel au moins une caméra de profondeur (40) est prévue dans le bras de rotation (55), dans la colonne verticale (52) ou dans l'unité de levage (53).

4. L'appareil de radiographie de l'une des revendications 1 à 3, dans lequel l'information de mouvement est un vecteur de mouvement de deux trames successives.

5. Une méthode de correction d'une radiographie médicale, la méthode comprenant :
la radiographie d'un sujet et l'obtention de données de radiographie médicale du sujet par un appareil de radiographie à rayons X, et, à la même période, l'imagerie du sujet et l'obtention de données de profondeur du sujet par une caméra de profondeur (S10) ;
calculer une information de mouvement du sujet en comparant deux trames voisines des données de profondeur, et corriger les données de la radiographie médicale correspondant à la trame actuelle des données de profondeur en utilisant l'information de mouvement calculée par une unité de correction (S20) ; et
en effectuant un algorithme de reconstruction basé sur les données de la radiographie médicale corrigée,
**caractérisé en ce que** la méthode comprend en outre :
la composition d'une image de surface en profondeur du sujet en utilisant les données de profondeur et les données de la radiographie médicale par une unité de composition,
où le calcul d'une information de mouvement comprend :
la définition d'un N-1ème cadre, où N est un nombre entier, des données de profondeur comme cadre de référence (S201) ;
l'obtention des informations de mouvement du sujet en comparant le N-1ème cadre de référence des données de profondeur à un Nème cadre actuel de celui-ci (S202) ;
déterminer si les informations de mouvement du sujet dépassent un seuil prédéterminé (S203) ; et
corriger les données d'une radiographie médicale correspondante en appliquant les informations de mouvement à une formule de transformation affine lorsque les informations de mouvement dépassent le seuil prédéterminé (S204).
